# EUROPEAN PATENT APPLICATION

(11) **EP 2 952 109 A1**
(43) Date of publication of application: **09.12.2015**
(21) Application number: 14177955.3
(22) Date of filing: 22.07.2014
(51) Int. Cl.: A24F 47/00, B05B 7/16, H01M 2/30, A61M 15/06

(54) **Atomizer for electronic cigarette**

(30) Priority: 06.06.2014 CN 201420298335 U
(71) Applicant: Huang, Jinzhen, Shenzhen, Guangdong (CN)
(72) Inventor: Huang, Jinzhen, Shenzhen, Guangdong (CN)
(74) Representative: Sun, Yiming

(57) **Abstract**

The present application is directed to electronic cigarette accessories, providing an atomizer for electronic cigarette, including a heating wire, an atomizing cup, an atomizing chamber and four ports, wherein the two ports are voltage input ports and another two ports are expansion ports. The atomizer for electronic cigarette in the present application includes two voltage input ports and two expansion ports. The expansion ports can act as input ports of the external information or voltage input ports.

## Description

### FIELD OF THE TECHNOLOGY

The present application is directed to electronic cigarette accessories, in particular, to an atomizer for electronic cigarette.

### BACKGROUND

At present, the use of electronic cigarette as a real cigarette replacement allows the possession of safe, environmental-friendly, and harmless without second-hand smoke as such beneficial characteristics and it has already been widely applied by smokers. The electronic cigarette is provided with heating wires which must have electrode being the input port in order to connect with the battery of the electronic cigarette.

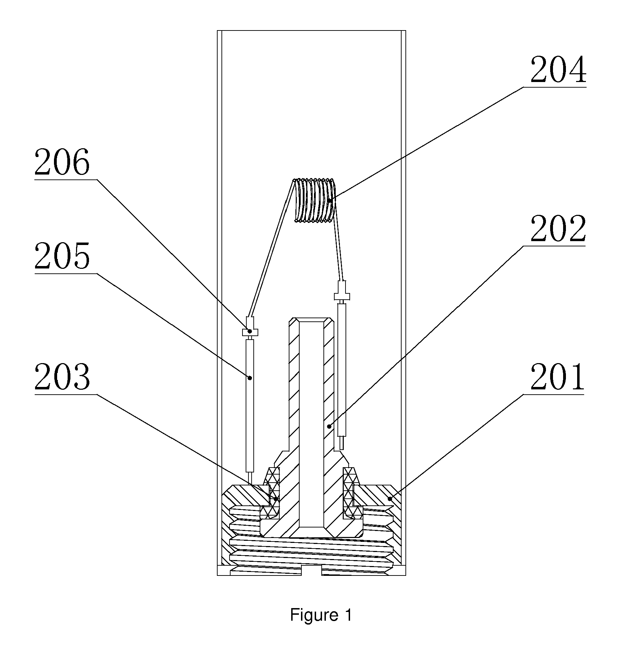

Figure 1 illustrates a common atomizer for electronic cigarette. Two poles of the common atomizer are formed from an external spiral ring 201 and an internal gas leading tube 202, while the center is insulated by an insulation circle 203. A heating wire 204 and a wire 205 are connected through a rivet 206. Such atomizer only has two input ports that heat the heating wires 204, and does not have expansion capability. Furthermore, components forming the two poles of the atomizer need to apply sophisticated lathe in order to be produced from the processing. Therefore, the production craft is complicated and the cost is very expensive.

### SUMMARY OF THE INVENTION

The technical problem that the present application needs to solve is to provide an atomizer for electronic cigarette that possesses an expansion capability.

The present application is directed to an atomizer for electronic cigarette, including a heating wire, an atomizing cup and an atomizing chamber, wherein the atomizer for electronic cigarette further includes four ports, two ports are voltage input ports and another two ports are expansion ports.

The expansion ports may be input ports of sensors or voltage input ports.

The expansion ports may be the voltage input ports, and the atomizer for electronic cigarette may include two strands of heating wires, two pairs of voltage input ports electrically connect with the two strands of heating wires, respectively.

The atomizer for electronic cigarette may further include a heating wire securing base that connects with the atomizing chamber, and the heating wire securing base may include four holes, the four ports are four electrodes, the four electrodes are embedded inside the four holes, respectively.

The atomizing chamber may include a securing base sealing pad, an external tube wall, an oil control plug and an upper cap, the atomizing chamber is formed from the surrounding of the securing base sealing pad, the external tube wall, the oil control plug and the upper cap.

The atomizer for electronic cigarette according may further include a gap on the external tube wall, wherein the oil control plug fills inside a part of a space of the gap, and the oil control plug and the gap form an oil leading hole.

The atomizer for electronic cigarette may further include an oil leading rope within the atomizing chamber, wherein one end of the oil leading rope passes through the oil leading hole and enters inside the atomizing cup and another end or a center portion of the oil leading rope lies in proximity to the heating wires.

The atomizer for electronic cigarette may further include a through hole on the upper cap, wherein the atomizing chamber connects with an external area through the through hole.

The atomizer for electronic cigarette may further include an atomizing cup connecting base, the atomizing cup connecting base is installed on the external tube wall, a top part of the atomizing cup connecting base connects with the atomizing cup.

The atomizing cup connecting base and the external tube wall may have inter-nested connection, the atomizer for electronic cigarette may further include an atomizing cup sealing circle embedded in a connection site of the atomizing cup connecting base and the external tube.

In comparing the present application with existing technology, the beneficial effects lie in: the atomizer for electronic cigarette in the present application includes two voltage input ports and two expansion ports, the expansion ports can act as input ports or voltage input ports of external information.

### BRIEF DESCRIPTION OF THE DRAWINGS

Figure 1 is longitudinal sectional illustrative view of an atomizer for electronic cigarette in the prior art.
Figure 2 is a front illustrative view of an atomizer for electronic cigarette under an embodiment in the present application.
Figure 3 is a top illustrative view of Figure 2.
Figure 4 is a bottom illustrative view of Figure 2.
Figure 5 is a sectional illustrative view of Figure 2 along the C-C sectional line.
Figure 6 is a sectional illustrative view of Figure 4 along the D-D sectional line.

### DETAILED DESCRIPTION OF THE INVENTION

In order to enable the technical problem, technical solution and beneficial effect that the present application seeks to solve to be clearer in understanding, below is a further detailed description of the application with combination of the drawings and embodiments. It should be understood that the specific embodiments described merely serve to explain the present application and is not in any way used to limit the same.

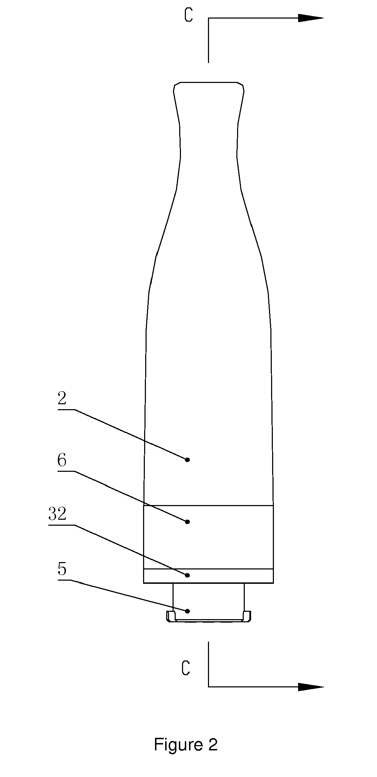

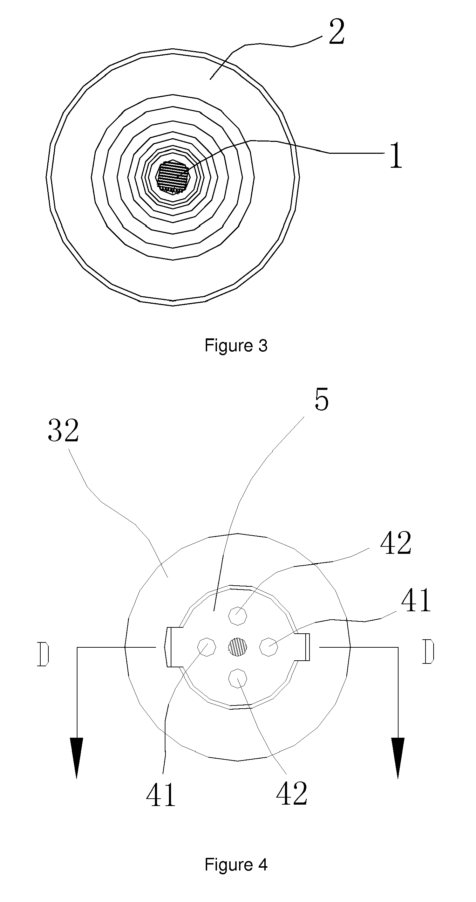

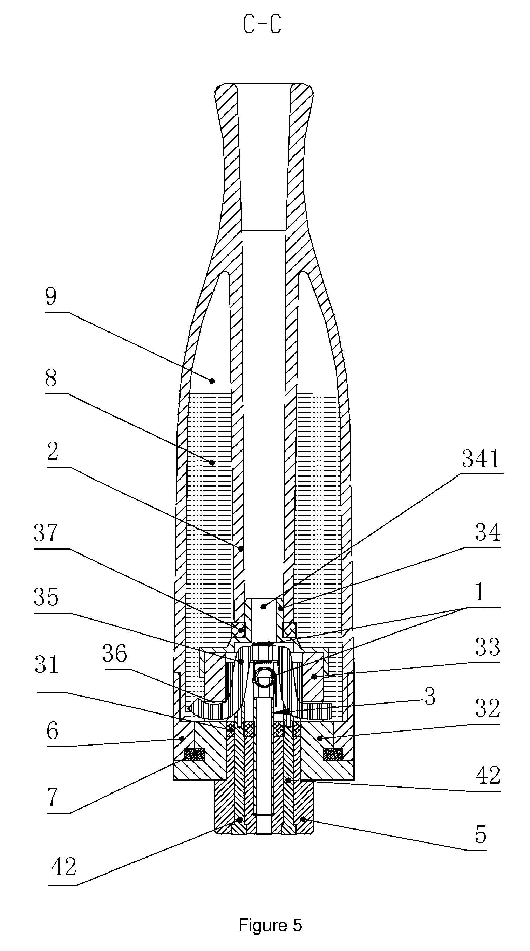

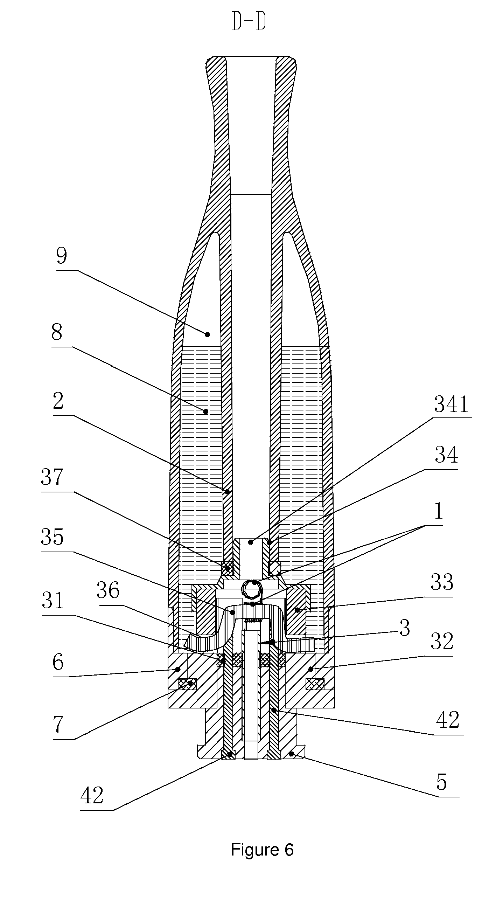

Figures 2 to 6 illustrate an embodiment of an atomizer for electronic cigarette in the present application which includes a heating wire 1, an atomizing cup 2, an atomizing chamber 3 and four ports, wherein two ports are the voltage input ports 41 and another two ports are the expansion ports 42.

The above expansion ports 42 can be input ports of sensors. The sensors can be thermocouple sensors, such that the temperature of the heating wire 1 can be monitored, for realizing the atomization of oil under a safe temperature. Obviously, the expansion port 42 can act as an input port of other external information. Furthermore, the expansion port 42 can also be voltage input port. This way, the two pairs of electrodes of voltage input ports each electrically connect to one thread of heating wire 1, so as to realize the work of the dual heating wires 1, allowing the smoke amount produced by the atomizer for electronic cigarette to double relative to the general atomizers.

Specifically, the above atomizer for electronic cigarette also includes a heating wire securing base 5 that connects to the atomizing chamber 3. The heating wire securing base 5 is provided with four holes (not shown in the drawings). The four ports are the four electrodes. The four electrodes are respectively embedded within the four holes. In particular, the two electrodes of the voltage input port 41 directly connect to the heating wire 1, clamp pressuring can be applied to allow the two electrodes to combine with the heating wires 1. Relative to the connection method of existing electronic cigarette, the present application eliminates the wires and rivets in between, while the structure and production craft are simple. No welding and wires as such processes to composite impurity materials are not necessary, so that it is more environmental friendly.

The atomizing chamber 3 includes a securing base sealing pad 31, an external tube wall 32, an oil control plug 33, an upper cap 34 and an oil leading rope 35. The atomizing chamber 3 is formed from the surrounding of the securing base sealing pad 31, external tube wall 32, oil control plug 33 and upper cap 34. On the external tube wall 32 is provided with a gap (not shown in the drawings). The oil control plug 33 fills inside part of the space of the gap. The oil control plug 33 and the gap form an oil leading hole 36. One end of the oil leading rope 35 passes through the oil leading hole 36 and enters into the atomizing cup 2. Another end or the center portion lies in proximity to the heating wire 1. The upper cap 34 is provided with through holes 341. The external circumference of the upper cap 34 is encapsulated with a sealing circle 37. The atomizing chamber 3 connects to the external area through the through holes. The above mentioned oil control plug 33 is a silicone piece. The size and length of the oil leading hole 36 can be freely adjusted according to different viscosities of the oil, so as to ensure the evenness and consistency in the entering of the oil.

The above atomizer for electronic cigarette includes an atomizing cup connecting base 6. The atomizing cup connecting base 6 is installed on the external tube wall 32, whose top part is connected with the atomizing cup 2. The atomizing cup connecting base 6 and the external tube wall 32 have inter-nested connection, and the connecting site of the two is embedded with an atomizing cup sealing circle 7.

Under normal condition, within the atomizing cup 2 is stored with a certain volume of oil 8 and small amount of air 9. The pressure produced from the liquid oil 8 and the pressure of the air 9 at the inner upper part of the atomizing cup 2 take effect on the oil leading holes 36. Since the oil leading holes 36 are obstructed by the oil leading rope 35, and with the effect of the atmospheric pressure inside the atomizing cup 2, the oil 8 inside the atomizing cup 2 is tightly sealed inside the atomizing cup 2.

When the atomizer for electronic cigarette is at work, the air inside the atomizing chamber 3 is sucked outside through the through holes 341. The barometric pressure of the atomizing chamber 3 is reduced, while the oil 8 of the atomizing cup 2 leaks out from the oil leading holes 36 under the double effect of the pressure of the oil in itself and the pressure of the air 9 at the inner upper part of the atomizing cup 2, and penetrates in the heating wires 1 along the oil leading rope 35. After the heating wire 1 is electrified, energized atomized oil 8 is produced and forms smoke. In such process, the air inside the atomizing chamber 3 would be incessantly sucked out through the through holes 341. The heating wire 1 is electrified and produces smoke, and continuously consumes the oil 8 of the atomizing cup 2. The pressure of the oil 8 inside the atomizing cup 2 and the pressure of the air 9 at the inner upper part are reduced. When such pressure is less than the atmospheric pressure of the atomizing chamber 3 to a certain value, the air would penetrate to the inner upper part of the atomizing cup 2 through the oil leading holes 36, until the pressure of the air 9 at the inner upper part of the atomizing cup 2 and the pressure of the oil 8 in itself are balanced with the external pressure. In such process, the oil leading holes 36 and the oil leading rope 35 form a gas and liquid exchange system. The present embodiment applies the pressure sealing concept to store the electronic oil, and eliminates the traditional chemical fiber foam for oil storing. Therefore, the storage effect of the oil 8 becomes more environmental friendly and hygienic.

The above is merely a preferred embodiment of the present application, and does not serve to limit the subject application. Any amendments, equivalent replacements and modifications etc. that are within the spirit and concept of the present application should all fall within the scope of protection of the subject application.

## Claims

1. An atomizer for electronic cigarette, comprising a heating wire, an atomizing cup and an atomizing chamber, wherein the atomizer for electronic cigarette further comprises four ports, two ports are voltage input ports and another two ports are expansion ports.

2. The atomizer for electronic cigarette according to claim 1, wherein the expansion ports are input ports of sensors or voltage input ports.

3. The atomizer for electronic cigarette according to claim 2, wherein the expansion ports are the voltage input ports, the atomizer for electronic cigarette comprises two strands of heating wires, two pairs of voltage input ports electrically connect with the two strands of heating wires, respectively.

4. The atomizer for electronic cigarette according to claim 1, wherein the atomizer for electronic cigarette further comprises a heating wire securing base that connects with the atomizing chamber, the heating wire securing base comprises four holes, the four ports are four electrodes, the four electrodes are embedded inside the four holes, respectively.

5. The atomizer for electronic cigarette according to claim 1, wherein the atomizing chamber comprises a securing base sealing pad, an external tube wall, an oil control plug and an upper cap, the atomizing chamber is formed from the surrounding of the securing base sealing pad, the external tube wall, the oil control plug and the upper cap.

6. The atomizer for electronic cigarette according to claim 5, further comprising: a gap on the external tube wall, wherein the oil control plug fills inside a part of a space of the gap, and the oil control plug and the gap form an oil leading hole.

7. The atomizer for electronic cigarette according to claim 6, further comprising an oil leading rope within the atomizing chamber, wherein one end of the oil leading rope passes through the oil leading hole and enters inside the atomizing cup and another end or a center portion of the oil leading rope lies in proximity to the heating wires.

8. The atomizer for electronic cigarette according to claim 5, further comprising: a through hole on the upper cap, wherein the atomizing chamber connects with an external area through the through hole.

9. The atomizer for electronic cigarette according to claim 5, wherein the atomizer for electronic cigarette further comprises an atomizing cup connecting base, the atomizing cup connecting base is installed on the external tube wall, a top part of the atomizing cup connecting base connects with the atomizing cup.

10. The atomizer for electronic cigarette according to claim 9, wherein the atomizing cup connecting base and the external tube wall have inter-nested connection, the atomizer for electronic cigarette further comprises an atomizing cup sealing circle embedded in a connection site of the atomizing cup connecting base and the external tube.
